# EUROPEAN PATENT APPLICATION

(11) **EP 4 588 906 A1**
(43) Date of publication of application: **23.07.2025**
(21) Application number: 23865829.8
(22) Date of filing: 13.09.2023
(51) Int. Cl.: C07C 17/06, C07C 25/125, C07C 45/30, C07C 45/82, C07C 49/80

(54) **METHOD FOR PRODUCING TEREPHTHALOYL CHLORIDE BY CONTROLLING LIGHT AMOUNT**

(30) Priority: 14.09.2022 KR 20220115697
(71) Applicant: Aekyung Chemical Co., Ltd., Seoul 04051 (KR)
(72) Inventor: KIM, Byung Jo, Daejeon 34023 (KR); SHIN, Chul, Seoul 08202 (KR); LEE, Ho Chang, Daejeon 34061 (KR); KIM, Suhan, Daejeon 34087 (KR); KANG, Dong Yun, Daejeon 34116 (KR); OH, Seunghyun, Daejeon 34107 (KR)
(74) Representative: Bassil, Nicholas Charles
(86) International application number: PCT/KR2023/013684
(87) International publication number: WO 2024/058532

(57) **Abstract**

The present invention relates to a method for producing terephthaloyl chloride, and the purity of hexachloroparaxylene, which is an intermediate raw material, can be improved by adopting a photochlorination method instead of a radical initiator, and by controlling a light amount of light irradiation to less than 2000 LUX, the advantages of a low occurrence of side reactions of hexachloroparaxylene and a high yield are provided. In addition, the present invention provides a method for producing terephthaloyl chloride in which, by adopting a circulation system, chlorine gas is not discharged to the outside.

## Description

### [Technical Field]

The present disclosure relates to a method for producing terephthaloyl chloride by controlling a light amount.

### [Background Art]

Terephthaloyl chloride (TPC) is a white solid or colorless needle-shaped crystal and mainly used as a polymer monomer of poly (paraphenylene terephthamide) and polysulfonamide.

In addition, the terephthaloyl chloride has broad development application prospects such as applications in the fields of pesticides, pharmaceuticals, and the like, as an admixture of a high molecular polymer.

Monomers mainly used in the production of the terephthaloyl chloride are terephthalic acid (TPA) or paraxylene.

However, when terephthaloyl chloride is produced using the terephthalic acid as a raw material, toxic solvents such as highly toxic phosgene and methylene chloride are used, and it is difficult to perform treatment and purification processes of by-products produced in a reaction process.

In addition, when terephthaloyl chloride is produced using paraxylene as a raw material, an intermediate step of producing paraxylene into hexachloroparaxylene is comprised, and when a chlorine gas used in the intermediate step is not reacted and discharged to the outside, it may affect the human body and the environments.

In addition, the intermediate step proceeds as a radical reaction, and since impurities may affect a radical initiator added to the radical reaction, a problem may arise when high purity terephthaloyl chloride is produced.

### [Disclosure]

### [Technical Problem]

An object of the present disclosure is to provide hexachloroparaxylene having a very low impurity content, by introducing a light irradiation reaction instead of a radical initiator.

Another object of the present disclosure is to provide high purity hexachloroparaxylene with a low side reaction and high yield, by controlling a light amount used in the light irradiation reaction.

Another object of the present disclosure is to provide a method for synthesizing hexachloroparaxylene in which a chlorine gas is not discharged to the outside, by circulating an unreacted chlorine gas to different reactors without discharging it.

Another object of the present disclosure is to provide a method for producing hexachloroparaxylene which allows relatively high chlorine gas solubility to be maintained within an appropriate viscosity, by controlling a photoreaction temperature inside the reactor in producing hexachloroparaxylene.

Still another object of the present disclosure is to provide terephthaloyl chloride by reacting the produced hexachloroparaxylene and terephthalic acid.

### [Technical Solution]

In one general aspect, a method for producing hexachloroparaxylene by photoreacting paraxylene and a chlorine gas, wherein a light amount added to the photoreaction is less than 2,000 LUX, is provided.

According to an example embodiment of the present disclosure, in the method for producing hexachloroparaxylene, a plurality of reactors may be connected in parallel.

According to an example embodiment of the present disclosure, the plurality of reactors may be reactors in which a first reactor and a second reactor are connected in parallel; the reaction may be started by continuously adding a chlorine gas to the first reactor filled with paraxylene to perform the reaction while discharging a hydrochloric acid gas as a by-product from conversion of hexachloroparaxylene to the outside; at a conversion rate of hexachloroparaxylene at which the chlorine gas continuously added to the first reactor where the reaction has been initiated starts to be discharged as an unreacted material, an unreacted chlorine gas, which is continuously added to the first reactor and does not participate in the reaction, may be transferred to the second reactor without being discharged to the outside to initiate a first reaction with paraxylene filled into the second reactor; the reaction in the first reactor may be terminated and the chlorine gas continuously added to the first reactor may be connected to the second reactor to start continuous addition to the second reactor; in a state in which the first reactor from which a product has been discharged is filled with paraxylene, at a conversion rate of hexachloroparaxylene at which the chlorine gas continuously added to the second reactor starts to be discharged as an unreacted material, the unreacted chlorine gas may be transferred to the first reactor to initiate a first reaction with paraxylene of the first reactor; and the process may be repeated, thereby producing hexachloroparaxylene without discharging the unreacted chlorine gas to the outside.

In another general aspect, a method for producing terephthaloyl chloride by reacting hexachloroparaxylene and terephthalic acid comprises: injecting a chlorine gas to paraxylene to produce hexachloroparaxylene; and reacting the hexachloroparaxylene and terephthalic acid in the presence of a Lewis acid catalyst to produce terephthaloyl chloride, wherein a light amount added to the producing of hexachloroparaxylene is less than 2,000 LUX.

According to an example embodiment of the present disclosure, in the method for producing hexachloroparaxylene, a plurality of reactors may be connected in parallel.

According to an example embodiment of the present disclosure, the plurality of reactors may be reactors in which a first reactor and a second reactor are connected in parallel; the reaction may be started by continuously adding a chlorine gas to the first reactor filled with paraxylene to perform the reaction while discharging a hydrochloric acid gas as a by-product from conversion of hexachloroparaxylene to the outside; at a conversion rate of hexachloroparaxylene at which the chlorine gas continuously added to the first reactor where the reaction has been initiated starts to be discharged as an unreacted material, an unreacted chlorine gas, which is continuously added to the first reactor and does not participate in the reaction, may be transferred to the second reactor without being discharged to the outside to initiate a first reaction with paraxylene filled into the second reactor; the reaction in the first reactor may be terminated and the chlorine gas continuously added to the first reactor may be connected to the second reactor to start continuous addition to the second reactor; in a state in which the first reactor from which a product has been discharged is filled with paraxylene, at a conversion rate of hexachloroparaxylene at which the chlorine gas continuously added to the second reactor starts to be discharged as an unreacted material, the unreacted chlorine gas may be transferred to the first reactor to initiate a first reaction with paraxylene of the first reactor; and the process may be repeated, thereby producing hexachloroparaxylene without discharging the unreacted chlorine gas to the outside.

According to an example embodiment of the present disclosure, the produced hexachloroparaxylene may further comprise a purification step.

According to an example embodiment of the present disclosure, the purification step may be performed by a distillation tower.

According to an example embodiment of the present disclosure, the distillation tower may purify hexachloroparaxylene at a bottom temperature of a distillation temperature of 100 to 300°C, a top temperature of a distillation temperature of 200 to 250°C, and a pressure of 1 to 20 torr.

According to an example embodiment of the present disclosure, the Lewis acid catalyst may be any one or two or more selected from aluminum trichloride, zinc chloride, and ferric trichloride.

According to an example embodiment of the present disclosure, the produced terephthaloyl chloride may further comprise a purification step.

According to an example embodiment of the present disclosure, the purification step may be performed by a distillation tower.

According to an example embodiment of the present disclosure, the distillation tower may purify hexachloroparaxylene at a controlled distillation temperature of 75 to 250°C and a pressure of 1 torr to 20 torr.

### [Advantageous Effects]

The present disclosure provides hexachloroparaxylene having a low impurity content, by providing hexachloroparaxylene by light irradiation instead of a radical initiator.

In addition, the present disclosure has low occurrence of a side reaction of hexachloroparaxylene, by controlling a light amount of light irradiation to less than 2000 LUX.

In addition, the present disclosure may maintain a relatively high chlorine gas solubility within appropriate viscosity to provide hexachloroparaxylene in a high yield, by dividing a reaction temperature into multiple sections and controlling the temperature during the photoreaction and gradually increasing the reaction temperature in each section.

The present disclosure may produce hexachloroparaxylene without discharging toxic chlorine gas to the outside, by introducing a chlorine gas circulation system which circulates an unreacted chlorine gas to the first reactor and the second reactor during production of hexachloroparaxylene as an intermediate raw material.

### [Description of Drawings]

FIG. 1 is a schematic diagram of a production step of producing terephthaloyl chloride.
FIG. 2 is a schematic diagram of a production step of hexachloroparaxylene.

### [Best Mode]

Hereinafter, the present disclosure will be described in more detail with reference to specific examples and example embodiments comprising the accompanying drawings. However, the following specific examples or example embodiments are only a reference for describing the present disclosure in detail, and the present disclosure is not limited thereto, and may be implemented in various forms.

In addition, unless otherwise defined, all technical terms and scientific terms have the same meanings as those commonly understood by one of those skilled in the art to which the present disclosure pertains. The terms used herein are only for effectively describing a certain specific example and are not intended to limit the present disclosure.

In addition, the singular form used in the specification and claims appended thereto may be intended to comprise a plural form also, unless otherwise indicated in the context.

In addition, unless particularly described to the contrary, "comprising" any elements will be understood to imply further inclusion of other elements rather than the exclusion of any other elements.

In addition, a chlorinated intermediate product described later in some embodiments of the disclosed technology may be monochloroparaxylene, dichloroparaxylene, trichloroparaxylene, tetrachloroparaxylene, or pentachloroparaxylene.

Conventionally, hexachloroparaxylene was provided by adding a radical initiator, but due to impurities therefrom, it was difficult to provide hexachloroparaxylene with a high purity.

In order to solve the problem, a mixture of paraxylene and a chlorine gas was irradiated with light to produce hexachloroparaxylene, but side reactions often occurred and the yield of hexachloroparaxylene was low.

Thus, in some embodiments of the disclosed technology, the problem was solved by providing a method of producing hexachloroparaxylene by reacting paraxylene and chlorine, wherein a light amount added to the reaction is less than 2,000 LUX.

The hexachloroparaxylene is produced by mixing paraxylene and a chlorine gas and irradiating the mixture with UV light. A light amount conventionally added to light irradiation was 2,000 LUX or more, but within the light amount range, a side reaction may occur and the yield of hexachloroparaxylene is low.

By irradiating the chlorine gas with UV, a chlorine radical is generated, and the generated chlorine radical is reacted with hydrogen of a methyl group by substitution to produce hexachloroparaxylene.

Conventionally, the reaction proceeded by irradiation with a light in a light amount of at least 2,000 LUX or more, at most 30,000 LUX or more, but within the light amount range, a side reaction occurs and the yield of hexachloroparaxylene is low.

Thus, the present inventors found that hexachloroparaxylene may be produced even when the irradiated light amount range is controlled to less than 2,000 LUX, 1,900 LUX or less, 1,800 LUX or less, 1,700 LUX or less, 1,500 LUX or less and 100 LUX or more, 200 LUX or more, 300 LUX or more, 400 LUX or more, 500 LUX or more, preferably 100 to 1,900 LUX, preferably 100 to 1,800 LUX, and rather, when hexachloroparaxylene is produced within the above light amount range, fewer side reactions occurs and the yield is high.

In addition, when the reaction occurs in the light amount range, reaction heat generated by the reaction is low, so that it is easy to perform temperature control and simultaneously a reaction rate may also be controlled.

In addition, though a light irradiation area is not limited as long as the reaction proceeds well, when it is specifically 30 cm² or less, preferably 20 cm² or less, and more preferably 10 cm² or less, the reaction may proceed better.

In addition, as a light source used in the light irradiation reaction used in some embodiments of the disclosed technology, an LED lamp is used.

Conventionally, a mercury lamp was used, but in the case of the mercury lamp, a short wavelength light from a low pressure mercury lamp may cause another photochemical side reaction to lower the purity of a product, and a long wavelength light from a high pressure mercury lamp is insufficient to cause a chlorine radical reaction, so that more energy may be consumed. In addition, when the mercury lamp is used, more heat is produced, and a problem may arise, for example, a cooling device should be additionally introduced in order to lower the heat.

Thus, the disadvantage of the mercury lamp has been overcome by using an LED lamp in some embodiments of the disclosed technology. By using the LED lamp, a wavelength appropriate for the reaction may be controlled to lower a photochemical side reaction, and since heat occurs less even for a long-term use as compared with a mercury lamp, an additional cooling device is not needed. In addition, since the LED lamp has low power consumption, energy efficiency is high.

Hydrochloric acid gas (HCl) is discharged as a by-product through the photoreaction.

However, some unreacted chlorine gas is also mixed with the hydrochloric acid gas by the light irradiation reaction and discharged together. In particular, as the light irradiation reaction proceeds, a reaction rate of the chlorine gas is lowered by steric effects, so that the content of the unreacted chlorine gas discharged to the outside is increased.

Thus, the present disclosure suggests a novel method for producing hexachloroparaxylene which may consume all of the unreacted chlorine gas without discharging it to the outside.

In the novel method for producing hexachloroparaxylene, a plurality of reactors are connected in parallel and an unreacted chlorine gas discharged sequentially from the plurality of reactors is transferred to another reactor and circulated.

In addition, the number of plural reactors may be 2 or 3 or more, but is not limited thereto.

Hereinafter, detailed description will be provided using the reactors in which two or more reactors are connected in parallel.

In some embodiments of the disclosed technology, a reaction is started by continuously adding a chlorine gas to the first reactor filled with paraxylene to perform the reaction while discharging a hydrochloric acid gas as a by-product from conversion of hexachloroparaxylene to the outside. Herein, the added chlorine gas is consumed by quantitative reaction with paraxylene. Subsequently, at a conversion rate of hexachloroparaxylene at which the chlorine gas continuously added to the first reactor starts to be discharged as an unreacted material, when an unreacted chlorine gas, which is continuously added to the first reactor and does not participate in the reaction, is discharged from the first reactor, the discharged unreacted chlorine gas is transferred to the second reactor without being discharged to the outside to initiate a first reaction with paraxylene filled into the second reactor. The circulated chlorine gas is stopped from being introduced to the first reactor as soon as the reaction of the first reactor is terminated, and the chlorine gas introduced to the first reactor is connected to the second reactor and continuously introduced to the second reactor to participate in the reaction.

Subsequently, in a state in which the first reactor from which a product has been discharged is filled with paraxylene again, at a conversion rate of hexachloroparaxylene at which the chlorine gas continuously added to the second reactor starts to be discharged as an unreacted material, the unreacted chlorine gas is circulated to the first reactor to initiate a first reaction with paraxylene of the first reactor; and the process is repeated, thereby producing hexachloroparaxylene without discharging the unreacted chlorine gas to the outside.

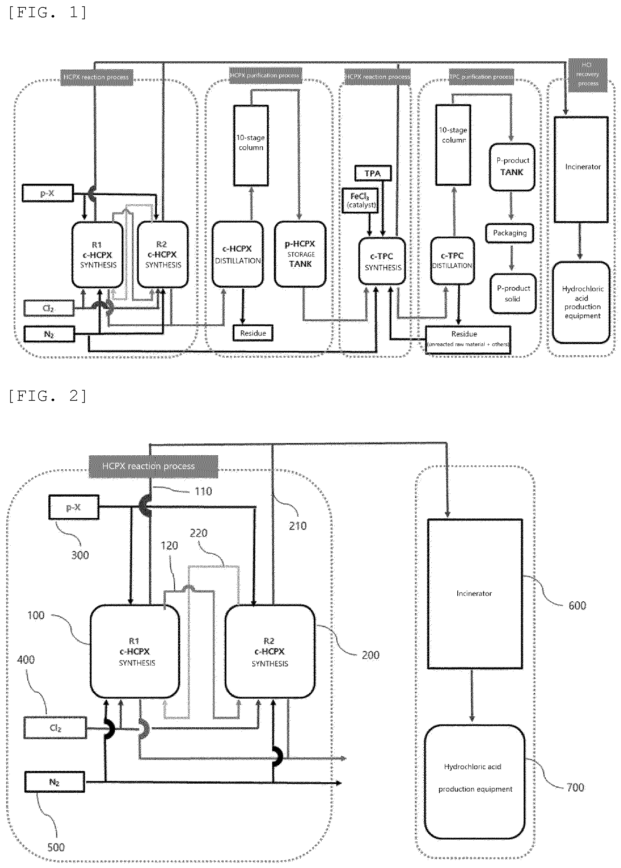

The method for producing hexachloroparaxylene will be described in more detail by dividing the method into steps with reference to the following FIG. 2.

According to an example embodiment, production steps of the hexachloroparaxylene may comprise:
S1) adding paraxylene to a first reactor 100 and a second reactor 200 and filling the reactors with paraxylene;
S2) continuously adding a chlorine gas to the first reactor 100, reacting the added chlorine gas with paraxylene 100%, transferring the chlorine gas as a by-product discharged from the reaction to an incinerator 600 through a first transfer pipe 110, and recovering hydrochloric acid;
S3) blocking the first discharge transfer pipe 110 when the reaction of the chlorine gas in the first reactor 100 is decreased to 100% or less to produce an unreacted chlorine gas;
S4) after blocking the first discharge transfer pipe 110, transferring the unreacted chlorine gas and a remaining hydrochloric acid gas in the first reactor 100 to the second reactor 200 through the first cross transfer pipe 120, performing a reaction first in the second reactor 200, producing hexachloroparaxylene in the first reactor 100 and the second reactor 200 simultaneously;
S5) transferring a hydrochloric acid gas produced as a by-product in the second reactor 200 to the incinerator 600 through a second discharge transfer pipe 210 in an upper portion of the second reactor 200;
S6) when the reaction in the first reactor 100 is terminated, blocking the first cross transfer pipe 120 and changing addition of the chlorine gas which has been added to the first reactor 100 to the second reactor 200;
S7) discharging hexachloroparaxylene as a product in the first reactor 100 and adding new paraxylene;
S8) when a chlorine gas reaction in the second reactor 200 is decreased to 100% or less to produce the chlorine gas, blocking the second discharge transfer pipe 210, adding an unreacted chlorine gas and a remaining hydrochloric acid in the second reactor 200 into the first reactor 100 through the second cross transfer pipe 220, and proceeding with a hexachloroparaxylene production reaction in the second reactor 200 and the first reactor 100 simultaneously;
S9) after the reaction of the second reactor 200 is terminated, adding the chlorine gas added to the second reactor 200 to the first reactor 100 to perform a reaction, opening the first discharge transfer pipe 110, transferring the hydrochloric acid as a by-product to the incinerator 600, and recovering the hydrochloric acid; and
S10) discharging hexachloroparaxylene inside the second reactor 200 and adding new paraxylene, and may comprise repeating S2) to S10) to continuously produce hexachloroparaxylene.

In the production steps of hexachloroparaxylene, the chlorine gas and the paraxylene may be produced into hexachloroparaxylene through a photoreaction. The chlorine gas is decomposed into a chlorine radical by the photoreaction and the chlorine radical is substituted with hydrogen of a methyl group of paraxylene to produce hexachloroparaxylene, and hydrochloric acid is discharged as the by-product.

Since the chlorine gas may be all consumed in the reaction through the method for producing hexachloroparaxylene, the chlorine gas which is fatal to the human body or the environment may be prevented from being discharged to the outside.

In particular, the mixed gas of the chlorine gas and the hydrochloric acid gas which is added to the reactor through the cross transfer pipe has a high probability of causing a side reaction as compared with a single chlorine gas at a high light amount, and thus, a problem of reduced reactivity may arise.

Thus, in some embodiments of the disclosed technology, reactivity may rather lower the side reaction by irradiation with the light amount of less than 2,000 LUX, and thus, hexachloroparaxylene may be obtained in a high yield.

In addition, as the content of hexachloroparaxylene produced by the reaction is increased, viscosity is increased. Accordingly, when the temperature is raised in order to lower the viscosity, solubility of a chlorine gas inside liquid paraxylene is lowered to decrease a reaction rate.

Thus, the present disclosure improves the above problems by adjusting the reaction temperature to a plurality of temperature sections according to the conversion rate of hexachloroparaxylene.

Specifically, in some embodiments of the disclosed technology, the temperature section is divided into at least 3 step sections, 4 step sections, 5 step sections, 6 step sections, 7 step sections, 8 step sections, 9 step sections, or 10 or more step sections from the lowest temperature to the highest temperature and the reaction is performed while heating, thereby controlling a chlorine gas solubility while maintaining the viscosity to a constant level, and thus, a reaction rate is high, chlorine may participate in the reaction as much as possible without external leakage, and thus, a high yield of 95% or more may be achieved therefrom.

According to a specific example according to the present disclosure, the reaction is initiated and performed for 4 hours under the conditions of heating from 13°C to 20°C, and as the conversion rate proceeds, the reaction is performed at 47°C to 60°C for 4 hours, at 63°C to 67°C for 4 hours, at 67°C to 83°C for 14 hours, at 87°C to 99°C for 2 hours, and at 106°C to 113°C for 2 hours to convert the reactant into 95% or more, preferably 98% or more of a product, without external leakage of the chlorine gas.

In addition, since the reactor temperature is controlled to the temperature range under the photoreaction conditions, the reactivity may be maintained at a high level even with the use of the mixed gas of chlorine and hydrochloric acid.

The prepared hexachloroparaxylene according to an example embodiment of the present disclosure and terephthalic acid may be reacted to produce terephthaloyl chloride.

Specifically, the production steps of terephthaloyl chloride comprise: producing hexachloroparaxylene from paraxylene; purifying the produced hexachloroparaxylene; reacting the purified paraxylene and terephthalic acid in the presence of an acid catalyst to prepare terephthaloyl chloride; and purifying the produced terephthaloyl chloride.

First, the purifying step of the hexachloroparaxylene may comprise recrystallization, rectification, distillation, and the like, and the distillation may comprise distillation under vacuum, molecular distillation, or the like.

In the purification step, the distillation may be performed using a distillation tower, and the distillation tower is not limited as long as it is a common device for distilling a compound.

The distillation tower may purify hexachloroparaxylene under the conditions of a bottom temperature of a distillation temperature of 100 to 300°C, a top temperature of the distillation tower of 100 to 250°C, and a pressure of 1 to 20 torr, but is not limited thereto.

In addition, the catalyst used in the step of producing terephthaloyl chloride by reacting the purified hexachloroxylene with terephthalic acid in the presence of an acid catalyst may be an acid catalyst.

The acid catalyst may be a Lewis acid catalyst, and the Lewis acid catalyst may comprise any one or two or more selected from aluminum trichloride, zinc chloride, and ferric trichloride, but it is not limited as long as it is a catalyst commonly used in the synthesis.

In the synthesis of terephthaloyl chloride, 30 to 300 parts by weight, preferably 40 to 200 parts by weight, more preferably 45 to 130 parts by weight of the terephthalic acid with respect to 100 parts by weight of the hexachloroparaxylene may be added, but is not limited thereto.

In addition, 0.001 to 10 parts by weight, preferably 0.003 to 1 part by weight, and more preferably 0.005 to 0.01 parts by weight of the acid catalyst with respect to 100 parts by weight of the hexachloroparaxylene may be comprised, but is not limited thereto.

In addition, according to an example embodiment of the present disclosure, the produced terephthaloyl chloride may further comprise a purification step, the purification step of terephthaloyl chloride may comprise recrystallization, rectification, distillation, and the like, and the distillation method may comprise distillation under vacuum, molecular distillation, or the like.

In the purification step of terephthaloyl chloride, the distillation may be performed using a distillation tower, and the distillation tower is not limited as long as it is a common device for distilling a compound.

The distillation tower may purify terephthaloyl chloride at a controlled distillation temperature of 75 to 250°C and a pressure of 1 torr to 20 torr, but is not limited thereto.

Hereinafter, the present disclosure will be described in more detail with reference to the examples and the comparative examples. However, the following examples and comparative examples are only an example for describing the present disclosure in more detail, and do not limit the present disclosure in any way.

### [Example 1]

### 1) Production step of hexachloroparaxylene (hexachloro-p-xylene)

In a paraxylene supply part 300, 100 parts by weight of paraxylene was added to a first reactor 100 and a second reactor 200, respectively; in a nitrogen gas supply part 500, nitrogen was injected into the first reactor 100 to remove air inside; and in a chlorine gas supply part 400, a chlorine gas was injected to the first reactor 100 and photoreacted to produce hexachloroparaxylene.

The injected chlorine gas was injected at 30 parts by weight per hour with respect to 100 parts by weight of the paraxylene.

For the reaction temperature in the photoreaction, as the reaction proceeded in the first reactor 100, an intermediate product was produced according to the conversion rate of the following Table 1, the melting point of the intermediate produced mixture increased due to the production of the intermediate product, and the reaction proceeded at the temperature and for a period of time shown in Table 2 under multi-stage heating conditions. During the reaction, stirring was performed at 300 rpm, and the photoreaction was performed by irradiating a large area of 15 cm² per 1 kg of a raw material with 1,000 LUX of UV light.

In addition, an external chlorine gas added to the first reactor was consumed by quantitatively participating in the reaction at the beginning of the reaction, and the hydrochloric acid as a by-product at this time was discharged to an incinerator 600 through a first discharge transfer pipe 110. When the yield of hexachloroparaxylene reached 0.1%, an unreacted chlorine gas started to be discharged to the outside, and thus, the unreacted chlorine gas was circulated to the second reactor at this time to initiate the chlorination reaction in the second reactor under the same condition as the first reactor.

The reaction was performed under the conditions of Tables 1 and 2 and terminated after 32 hours, a chlorine gas introduction pipe of the first reactor was connected to a chlorine gas introduction pipe of the second reactor, and the chlorine gas was added to the second reactor. The reaction was performed under the conditions of the conversion rate, the reaction temperature, and the reaction time of the second reactor in Tables 3 and 4.

In the second reactor, when an unreacted chlorine gas occurred at a hexaparaxylene yield of 0.1%, the chlorine gas was transferred to the first reactor, and the chlorination reaction was initiated in the first reactor.

**[Table 1]**

| Reaction time lapse (H) | MP (°C) | |
|---|---|---|
| | Starting point (melting start point) | End point (melting end point) |
| 0 | 13 | |
| 2 | 13-20 | |
| 4 | | |
| 8 | 47 | 60 |
| 12 | 56 | 64 |
| 16 | 63 | 67 |
| 20 | 65 | 70 |
| 24 | 66 | 71 |
| 28 | 67 | 83 |
| 39 | 87 | 99 |
| 32 | 106 | 113 |

**[Table 2]**

| GC R.T | 2.63 | 4.92 | 5.83 | 7.33 | 8.24 | 8.53 | 8.92 | 9.27 | 9.49 |
|---|---|---|---|---|---|---|---|---|---|
| Reaction time (hr) | p-X | Cl | Cl | Cl | Cl | Cl | Cl | Cl | HCPX |
| | | 1 | 2 | 2* | 3 | 4 | 4* | 5 | |
| 2 | 65.57 | 29.98 | 0.65 | 2.15 | 0.16 | 0.11 | 0.01 | - | - |
| 4 | 35.67 | 51.38 | 2.37 | 8.29 | 0.67 | 0.08 | 0.02 | - | - |
| 8 | 1.53 | 37.64 | 8.76 | 39.64 | 9.57 | 0.15 | 0.40 | - | 0.10 |
| 12 | - | 6.98 | 6. 68 | 42.44 | 35.38 | 1. 67 | 4.46 | 0.20 | 0.20 |
| 16 | - | 1.11 | 1.12 | 20.08 | 46.74 | 4.61 | 21.90 | 2.85 | 0.26 |
| 17 | - | - | 0.20 | 10.70 | 45.71 | 5.68 | 30.67 | 5.10 | 0.32 |
| 20 | - | - | - | 1.53 | 23.52 | 6.21 | 47.57 | 17.91 | 1. 89 |
| 24 | - | - | - | 0.02 | 2.80 | 2.33 | 39.19 | 43.06 | 10.61 |
| 28 | - | - | - | - | 0.08 | - | 4.70 | 35.71 | 56.35 |
| 30 | - | - | - | 0.07 | 0.20 | 0.03 | 0.76 | 15.33 | 79.62 |
| 31 | - | - | - | 0.03 | - | 0.06 | 0.17 | 2.69 | 92.98 |
| 32 | - | - | - | - | - | 0.08 | 0.05 | - | 96.26 |
| Drain | | | | | | | | | |

### 2) Purification step of hexachloroparaxylene

Hexachloroparaxylene produced in the first reactor 100 and the second reactor 200 was transferred to the bottom of a first distillation tower. In order to purify the hexachloroparaxylene, the first distillation tower performed purification at a total pressure of the distillation tower of 5 torr, a bottom temperature of 160°C, a top temperature of 150°C, and a stirring speed of 300 rpm and fractionated highly purified hexachloroparaxylene and the hexachloroparaxylene residue, and then the purified hexachloroparaxylene was transferred to a first storage tank and stored.

### 3) Reaction step of terephthaloyl chloride

In the purification step of hexachloroparaxylene, high purity hexachloroparaxylene stored in a first storage tank was transferred to a third reactor, terephthalic acid and an iron chloride (FeCl₃) catalyst was added to the third reactor, and a reaction was performed for 5 hours to produce terephthaloyl chloride.

The reaction was performed by mixing 100 parts by weight of terephthalic acid and 0.05 parts by weight of FeCl₃ with respect to 100 parts by weight of high purity hexachloro-p-xylene, and the reaction conditions were at a temperature of 120°C and a normal pressure.

### 4) Purification step of terephthaloyl chloride

The terephthaloyl chloride produced in 3) Reaction step of terephthaloyl chloride was transferred to a second distillation tower and first purified. The purification conditions of the second distillation tower were at a total pressure of the distillation tower of 5 torr, a top temperature of 160°C, a bottom temperature of 130°C, and a stirring speed of 300 **rpm.** The highly purified terephthaloyl chloride was transferred to a second storage tank and stored.

Hereinabove, although the present disclosure has been described by specific matters, limited example embodiments, and drawings, they have been provided only for assisting the entire understanding of the present disclosure, and the present disclosure is not limited to the example embodiments, and various modifications and changes may be made by those skilled in the art to which the present disclosure pertains from the description.

Therefore, the spirit of the present disclosure should not be limited to the above-described example embodiments, and the following claims as well as all modifications equal or equivalent to the claims are intended to fall within the scope and spirit of the disclosure.

### [Detailed Description of Main Elements]

- 100:: First reactor
- 110:: First discharge transfer pipe
- 120:: First cross transfer pipe
- 200:: Second reactor
- 210:: Second discharge transfer pipe
- 220:: Second cross transfer pipe
- 300:: Paraxylene supply part
- 400:: Chlorine gas supply part
- 500:: Inert gas supply part
- 600:: Incinerator
- 700:: Hydrochloric acid production equipment

## Claims

1. A method for producing hexachloroparaxylene by photoreacting paraxylene and a chlorine gas, wherein a light amount added to the photoreaction is less than 2,000 LUX.

2. The method for producing hexachloroparaxylene of claim 1, wherein in the producing of hexachloroparaxylene, a plurality of reactors are connected in parallel.

3. The method for producing hexachloroparaxylene of claim 2, wherein the plurality of reactors are reactors in which a first reactor and a second reactor are connected in parallel; the reaction is started by continuously adding the chlorine gas to the first reactor filled with paraxylene to perform the reaction while discharging a hydrochloric acid gas as a by-product from conversion of hexachloroparaxylene to an outside of the plurality of reactors; at a conversion rate of hexachloroparaxylene at which the chlorine gas continuously added to the first reactor where the reaction has been initiated starts to be discharged as an unreacted material, an unreacted chlorine gas, which is continuously added to the first reactor and does not participate in the reaction, is transferred to the second reactor without being discharged to the outside to initiate a first reaction with paraxylene filled into the second reactor; the reaction in the first reactor is terminated and the chlorine gas continuously added to the first reactor is connected to the second reactor to start continuous addition to the second reactor; in a state in which the first reactor from which a product has been discharged is filled with paraxylene, at a conversion rate of hexachloroparaxylene at which the chlorine gas continuously added to the second reactor starts to be discharged as the unreacted material, the unreacted chlorine gas is transferred to the first reactor to initiate the first reaction with paraxylene of the first reactor; and the process is repeated, thereby producing hexachloroparaxylene without discharging the unreacted chlorine gas to the outside.

4. A method for producing terephthaloyl chloride by reacting hexachloroparaxylene and terephthalic acid, the method comprising:
injecting a chlorine gas to paraxylene to produce hexachloroparaxylene; and
reacting the hexachloroparaxylene and terephthalic acid in the presence of a Lewis acid catalyst to produce terephthaloyl chloride,
wherein a light amount added to the producing of hexachloroparaxylene is less than 2,000 LUX.

5. The method for producing terephthaloyl chloride of claim 4, wherein in the producing of hexachloroparaxylene, a plurality of reactors are connected in parallel.

6. The method for producing terephthaloyl chloride of claim 5, wherein the plurality of reactors are reactors in which a first reactor and a second reactor are connected in parallel; the reaction is started by continuously adding the chlorine gas to the first reactor filled with paraxylene to perform the reaction while discharging a hydrochloric acid gas as a by-product from conversion of hexachloroparaxylene to an outside of the plurality of reactors; at a conversion rate of hexachloroparaxylene at which the chlorine gas continuously added to the first reactor where the reaction has been initiated starts to be discharged as an unreacted material, an unreacted chlorine gas, which is continuously added to the first reactor and does not participate in the reaction, is transferred to the second reactor without being discharged to the outside to initiate a first reaction with paraxylene filled into the second reactor; the reaction in the first reactor is terminated and the chlorine gas continuously added to the first reactor is connected to the second reactor to start continuous addition to the second reactor; in a state in which the first reactor from which a product has been discharged is filled with paraxylene, at a conversion rate of hexachloroparaxylene at which the chlorine gas continuously added to the second reactor starts to be discharged as the unreacted material, the unreacted chlorine gas is transferred to the first reactor to initiate the first reaction with paraxylene of the first reactor; and the process is repeated, thereby producing hexachloroparaxylene without discharging the unreacted chlorine gas to the outside.

7. The method for producing terephthaloyl chloride of claim 4, further comprising purification of the hexachloroparaxylene.

8. The method for producing terephthaloyl chloride of claim 7, wherein the purification is performed by a distillation tower.

9. The method for producing terephthaloyl chloride of claim 8, wherein the distillation tower purifies the hexachloroparaxylene at a bottom distillation temperature of 100 to 300°C, a top distillation temperature of 100 to 250°C, and a pressure of 1 to 20 torr.

10. The method for producing terephthaloyl chloride of claim 4, wherein the Lewis acid catalyst is any one or two or more selected from aluminum trichloride, zinc chloride, and ferric trichloride.

11. The method for producing terephthaloyl chloride of claim 4, further comprising purification of the terephthaloyl chloride.

12. The method for producing terephthaloyl chloride of claim 11, wherein the purification is performed by a distillation tower.

13. The method for producing terephthaloyl chloride of claim 12, wherein the distillation tower purifies hexachloroparaxylene at a controlled distillation temperature of 75 to 250°C and a pressure of 1 torr to 20 torr.
